# EUROPEAN PATENT APPLICATION

(11) **EP 2 201 981 A1**
(43) Date of publication of application: **30.06.2010**
(21) Application number: 09015876.7
(22) Date of filing: 22.12.2009
(51) Int. Cl.: A61N 5/10, A61B 5/113

(54) **Radiotherapy system**

(30) Priority: 26.12.2008 JP 2008333280
(71) Applicant: Hitachi Ltd., Chiyoda-ku Tokyo 100-8280 (JP)
(72) Inventor: Umekawa, Toru, Tokyo 100-8220 (JP); Nagamine, Yoshihiko, Tokyo 100-8220 (JP); Sasaki, Toshie, Tokyo 100-8220 (JP); Kidani, Takao, Tokyo 100-8220 (JP); Akiyama, Hiroshi, Tokyo 100-8220 (JP)
(74) Representative: Strehl Schübel-Hopf & Partner

(57) **Abstract**

The invention provides a radiotherapy system that achieves highly-accurate respiratory-gated irradiation with less x-ray exposure by performing x-ray imaging only during the respiratory phases that are necessary for the respiratory-gated irradiation.

An external respiration monitor 300 externally monitors an external respiratory index of a patient. An x-ray source 200 and an x-ray detector 210, or internal-respiration observation devices, acquire x-ray images of the patient and monitor an internal respiratory index of the patient using the positions of internal body structures indicated by the acquired x-ray images. An x-ray imaging gating device 310 gates the x-ray imaging performed by the internal-respiration observation devices with the use of the external respiratory index monitored by the external respiration monitor 300 such that the x-ray imaging is performed while the external respiratory index is within a predetermined range. An irradiation gating device 410 gates the irradiation of a treatment beam with the use of the internal respiratory index monitored by the internal-respiration observation devices.

## Description

### 1. Field of the Invention

The present invention relates generally to radiotherapy systems that irradiate patients with treatment beams and particularly to respiratory-gated radiotherapy systems that radiate treatment beams in synchronization with particular phases of patients' respiratory cycles.

### 2. Description of the Related Art

In radiotherapy that involves the irradiation of x-rays, particle beams, or the like onto the body of a patient for treatment purposes, of importance for enhancing the treatment effects is highly accurate positioning of the irradiation target according to the treatment plan. Highly accurate positioning enables the reduction of the irradiation area margin that compensates for positioning-related uncertainties and results in the healthy tissues or organs that surround the irradiation target being less exposed to treatment beams.

When the target moves due to the respiratory motion of the patient, this requires a margin that allows for the respiratory motion. In order to reduce that margin, respiratory-gated irradiation is now employed, in which treatment beams are radiated only during particular phases of respiration. This reduces the required margin, for the respiratory motion is limited within the limited respiratory phases. There are two methods for observation respiratory phases: external observation and internal observation. External observation involves the use of externally observable indexes or parameters such as those indicating the movements of the body surface and respiratory flow rates. In contrast, internal observation involves the use of such indexes as indicate the positions of internal body structures (e.g., intracorporeal irradiation targets, bony structures, and diaphragm). The positions of such internal body structures can be obtained by x-ray imaging. Respiratory-gated irradiation methods that are based on internal observation are disclosed, for example, in Japanese Patent No. 3053389, JP-2008-154861-A, and JP-2004-283513-A.

### SUMMARY OF THE INVENTION

Internal observation enables highly accurate measurement of the positions of internal body structures since they are measured directly. However, x-ray exposure of the patient is inevitable during internal observation (i.e., x-ray imaging). Thus, it is desired that this x-ray exposure be as little as possible.

An object of the invention is therefore to provide a radiotherapy system that achieves highly-accurate respiratory-gated irradiation with less x-ray exposure by taking x-ray only during the respiratory phases that are necessary for the respiratory-gated irradiation.
1) To achieve the above object, a radiotherapy system according to the invention comprises: external-respiration observation means for externally monitoring an external respiratory index of a patient; internal-respiration observation means for acquiring x-ray images of the patient and monitoring an internal respiratory index of the patient using the positions of internal body structures indicated by the acquired x-ray images; imaging gating means for gating the x-ray imaging performed by the internal-respiration observation means with the use of the external respiratory index monitored by the external-respiration observation means such that the x-ray imaging is performed while the external respiratory index is within a predetermined range; and beam irradiation gating means for gating the irradiation of a treatment beam with the use of the internal respiratory index monitored by the internal-respiration observation means.
   By performing x-ray imaging only during the respiratory phases that are necessary for the respiratory-gated irradiation with the use of the above configuration, it is possible to achieve highly-accurate respiratory-gated irradiation with less x-ray exposure.
2) In the above radiotherapy system, the internal-respiration observation means preferably includes x-ray imaging control means for instructing the internal-respiration observation means to stop the x-ray imaging when a gating signal permitting treatment-beam irradiation which is generated by the beam irradiation gating means is turned off.
3) In the above radiotherapy system, the internal-respiration observation means preferably includes x-ray imaging control means for permitting the internal-respiration observation means to take the x-ray while a treatment beam can be radiated.
4) In the above radiotherapy system, the internal-respiration observation means preferably includes x-ray imaging control means for permitting the internal-respiration observation means to perform the x-ray imaging prior to a period during which a treatment beam can be radiated.
5) In the above radiotherapy system, the beam irradiation gating means preferably stores a condition for radiating a treatment beam so that the beam irradiation gating means can use the condition for later treatments.

In accordance with the invention, it is possible to achieve highly-accurate respiratory-gated irradiation with less x-ray exposure by performing x-ray imaging only during the respiratory phases that are necessary for the respiratory-gated irradiation.

### BRIEF DESCRIPTION OF THE DRAWINGS

FIG. 1 illustrates the configuration of a radiotherapy system according to a first embodiment of the invention;
FIG. 2 is a flowchart illustrating the operation of the radiotherapy system according to the first embodiment;
FIGs. 3A and 3B are timing charts illustrating the operation of the radiotherapy system according to the first embodiment;
FIGs. 4A and 4B are timing charts illustrating the operation of the radiotherapy system according to the first embodiment;
FIG. 5 is a flowchart illustrating the operation of a radiotherapy system according to a second embodiment of the invention;
FIGs. 6A to 6E are timing charts illustrating the operation of the radiotherapy system according to the second embodiment;
FIG. 7 is a flowchart illustrating the operation of a radiotherapy system according to a third embodiment of the invention;
FIGs. 8A to 8F are timing charts illustrating the operation of the radiotherapy system according to the third embodiment;
FIG. 9 is a flowchart illustrating the operation of a radiotherapy system according to a fourth embodiment of the invention; and
FIGs. 10A to 10F are timing charts illustrating the operation of the radiotherapy system according to the fourth embodiment.

### DESCRIPTION OF THE PREFERRED EMBODIMENTS

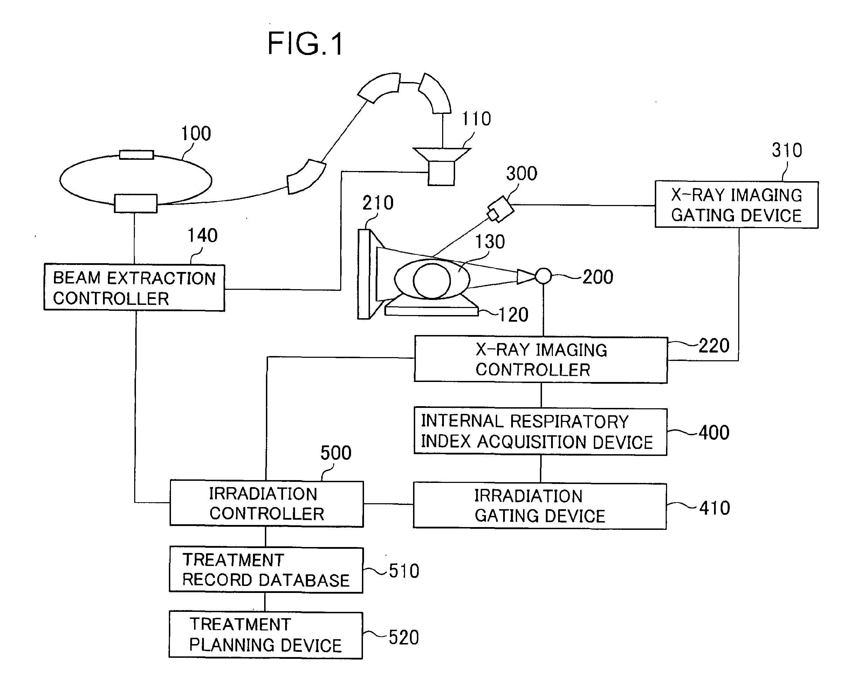

With reference to FIGs. 1 to 4B, the configuration and operation of a radiotherapy system according to a first embodiment of the invention will now be described. Described first is the configuration of the radiotherapy system of the first embodiment, which is shown in FIG. 1.

A beam accelerator 100 generates a treatment beam, and a nozzle 110 shapes the beam and radiates it onto a patient 130 on a couch 120. The beam accelerator 100 and the nozzle 110 are controlled by a beam extraction controller 140 upon beam irradiation. The beam extraction controller 140 performs its beam extraction control in response to a command from an irradiation controller 500. In response to a command from the operator, the irradiation controller 500 acquires from a treatment record database 510 a treatment plan that is created in advance by a treatment planning device 520. When the operator instructs the irradiation controller 500 to start a treatment, the irradiation controller 500 issues a beam extraction command to the beam extraction controller 140 according to the dose and method of beam irradiation specified by the plan of that treatment.

For the purpose of radiating treatment beams in synchronization with particular phases of the respiration of the patient 130, the radiotherapy system of the first embodiment is provided with two types of observation devices that observe the respiratory states of the patient 130: an external-respiration observation device and an internal-respiration observation device. The external-respiration observation device externally monitors measurable respiratory signals such as those changes in body shape, respiratory flow rates, and the like, and an external respiration monitor 300 serves as the external-respiration observation device. Signals obtained by the external respiration monitor 300 are input to an x-ray imaging gating device 310, where the signals are used for respiratory gating. The internal-respiration observation device observes respiratory phases based on the positional information of internal body structures such as intracorporeal treatment targets, bony structures, and diaphragm or of implanted markers. Used as the internal-respiration observation device is an x-ray imaging controller 220 that acquires x-ray images of the patient 130 with the use of an x-ray source 200 and an x-ray detector 210. The x-ray images acquired by the x-ray imaging controller 220 are sent to an internal respiratory index acquisition device 400 for analysis of respiratory indexes. The analyzed respiratory indexes are used for respiratory gating by an irradiation gating device 410.

Radiotherapy systems in general are equipped with x-ray devices for positioning patients, and the x-ray imaging device of the invention that comprises the x-ray source 200, the x-ray detector 210, and the x-ray imaging controller 220 can be used also as such a patient-positioning x-ray device. Positioning a patient is the step of conforming the positional relationship between the irradiation target and the treatment devices to the treatment plan, and x-ray images are used during the positioning step for the purpose of obtaining the positional information of internal body structures.

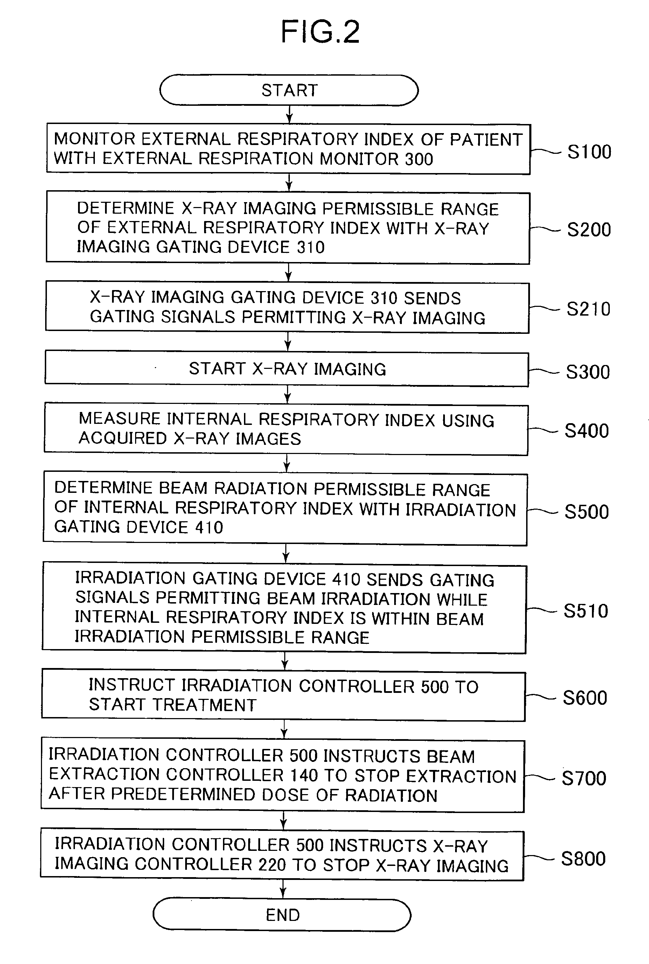

With reference now to FIGs. 2 to 4B, the operation of the radiotherapy system of the first embodiment is described.

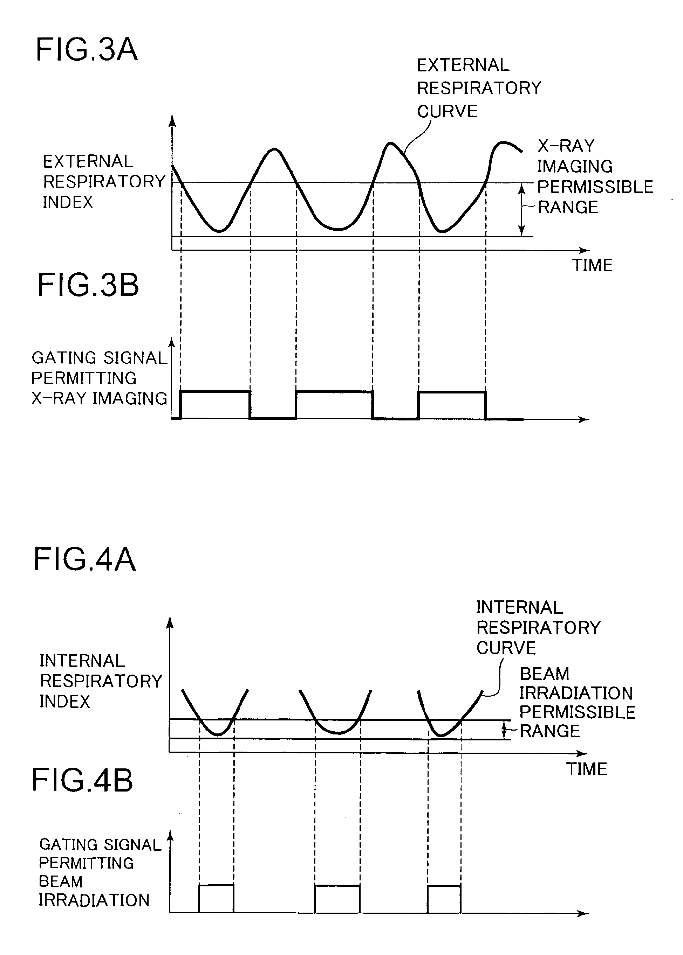

FIG. 2 is a flowchart illustrating the operation of the radiotherapy system of the first embodiment. FIGs. 3A to 3B and 4A to 4B are timing charts illustrating the operation of the radiotherapy system of the first embodiment.

In Step S100, the operator starts up the external respiration monitor 300 to start the monitoring of an external respiratory index of the patient 130. The monitored external respiratory index is input to the x-ray imaging gating device 310.

The monitoring of the external respiratory index is done by a laser rangefinder measuring positions on the body surface, a visible-light or infrared camera measuring the positions of markers placed on the body surface, or strain gauges placed on the body surface or a respiratory flow meter measuring respiratory flow rates. The external respiratory index as used herein refers to a measurable value that changes in response to respiration such as a position on the body surface. The external respiratory index is monitored during an entire treatment period.

In Step S200, the operator operates the x-ray imaging gating device 310 to set an x-ray imaging permissible range for the external respiratory index acquired by the external respiration monitor 300. The x-ray imaging gating device 310 is provided with means for the operator setting an x-ray imaging permissible range for the external respiratory index the x-ray imaging gating device 310 receives.

In Step S210, the x-ray imaging gating device 310 sends gating signals permitting x-ray imaging to the x-ray imaging controller 220.

Here, we briefly discuss an external respiratory curve with reference to FIG. 3A. As illustrated in FIG. 3A, an external respiratory curve is obtained by plotting the values of an external respiratory index against time.

The x-ray imaging gating device 310 is capable of displaying an external respiratory curve. By referring to the displayed external respiratory curve, the operator inputs into the x-ray imaging gating device 310 an x-ray imaging permissible range for the external respiratory index. While the external respiratory curve is within the x-ray imaging permissible range, the x-ray imaging gating device 310 sends gating signals permitting x-ray imaging to the x-ray imaging controller 220, as shown in FIG. 3B. It should be noted that the x-ray imaging permissible range can instead be set with the use of the change rates of the external respiratory curve. Also, the x-ray imaging gating device 310 can be allowed to store the x-ray imaging permissible range on a memory or a database for its later use as a default value.

With reference back to FIG. 2, in Step S300, the operator instructs the x-ray imaging controller 220 to start x-ray imaging. X-ray images are acquired intermittently while the x-ray imaging gating device 310 sends the gating signals permitting x-ray imaging to the x-ray imaging controller 220. One cycle of the external respiratory curve shown in FIG. 3A spans approximately 4 seconds although its length varies from person to person. Thus, the length of one cycle during which a gating signal permitting x-ray imaging is on, which is shown in FIG. 3B, is approximately 2 seconds. The acquired x-ray images are input to the internal respiratory index acquisition device 400.

In Step S400, the internal respiratory index acquisition device 400 analyzes the x-ray images acquired by the x-ray imaging controller 220 to obtain an internal respiratory index. The internal respiratory index is obtained from the positions of irradiation targets, bones, diaphragm, or implanted markers. The positional analysis of the irradiation targets and bones is done by acquiring x-ray images of relevant regions based on a treatment plan and searching for the best matched region or by the operator specifying a region in advance and obtaining the optical flow of that region by image matching. The positional analysis of the diaphragm exploits the fact that an intensity value of an x-ray image changes steeply at the position of the diaphragm. For example, the operator specifies an analysis region, and the diaphragm is located when the change rate of an intensity value within the analysis region exceeds a given value. Changes in the position of the diaphragm are then analyzed for obtaining the internal respiratory index. The positional analysis of the implanted markers is done by thresholding or pattern matching, using the fact that the markers on an x-ray image have high intensity values and distinctive shapes. The internal respiratory index obtained with the use of one or more of the above methods is sent to the irradiation gating device 410.

As stated above, x-ray images are acquired intermittently. Thus, when an analysis of the internal respiratory index needs to be followed by the next analysis without discontinuity at some later time, the radiotherapy system of the first embodiment can be provided with a mechanism for storing the on/off states of the last gating signal on a memory and resuming analysis from the last on or off state of that signal.

In Step S500, the operator inputs into the irradiation gating device 410 a beam irradiation permissible range for the internal respiratory index by referring to its internal respiratory curves displayed by the irradiation gating device 410. Similar to the x-ray imaging gating device 310 having means for setting an x-ray imaging permissible range for the external respiratory index, the irradiation gating device 410 is provided with means for accepting the input of a gating condition for treatment beam irradiation.

We now briefly discuss the internal respiratory curves with reference to FIG. 4A. As illustrated in FIG. 4A, the internal respiratory curves or waves are obtained by plotting the values of an internal respiratory index against time. The internal respiratory curves are discontinuous since x-ray imaging is gated.

Note that the set condition for permitting beam irradiation can be stored on a memory for use during later treatments in order to reduce X-ray exposure.

With reference back to FIG. 2, in Step S510, the irradiation gating device 410 sends such gating signals permitting beam irradiation as shown in FIG. 4B through the irradiation controller 500 to the beam extraction controller 140 while the internal respiratory index is within the beam irradiation permissible range.

With the above steps, respiratory-gated irradiation becomes ready, which is based on the gating signals permitting beam irradiation that are synchronous with the beam irradiation permissible periods of the internal respiratory index. As shown in FIGs. 3B and 4B, the period during which a gating signal permitting x-ray imaging is on is longer than the period during which a gating signal permitting beam irradiation is on. For the purpose of reducing x-ray exposure during x-ray imaging, the x-ray imaging permissible range can be adjusted such that the beam irradiation permissible periods coincide with the x-ray imaging permissible periods.

In Step S600, the operator instructs the irradiation controller 500 to start treatment.

In Step S700, the irradiation controller 500 instructs the beam extraction controller 140 to stop the beam extraction after a given dose of irradiation as specified by the treatment planning device 520.

Finally, in Step S800, the irradiation controller 500 instructs the x-ray imaging controller 220 to stop the x-ray imaging.

As stated above, the radiotherapy system of the first embodiment is designed to monitor an external respiratory index with the use of the external respiration monitor 300, and while the external respiratory index is within an x-ray imaging permissible range, the system sends gating signals permitting x-ray imaging to the x-ray imaging controller 220, thereby performing gated x-ray imaging. Thus, the system is capable of acquiring internal respiratory waves or curves necessary for the gating control of beam irradiation with less x-ray exposure.

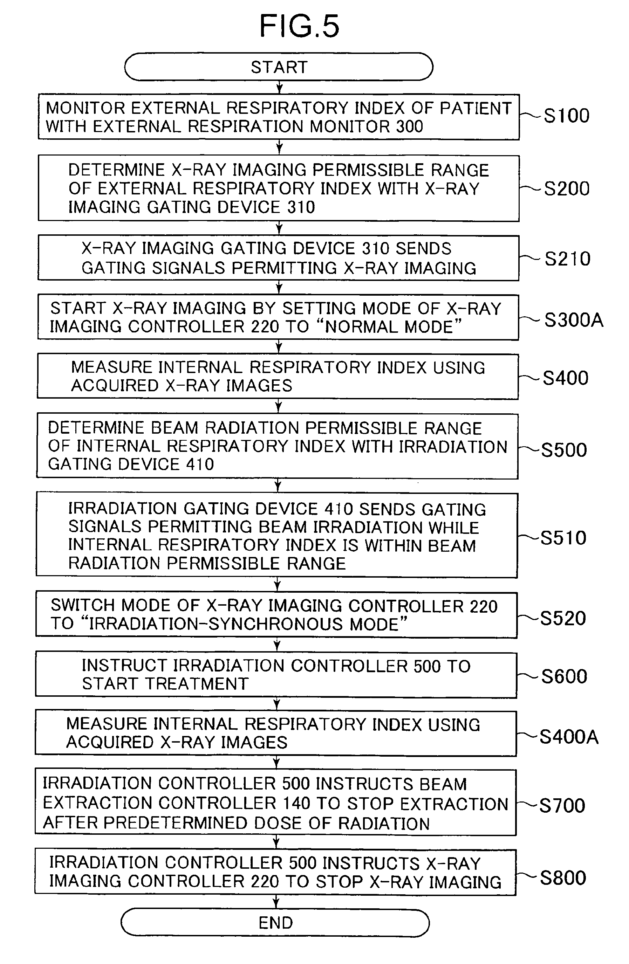

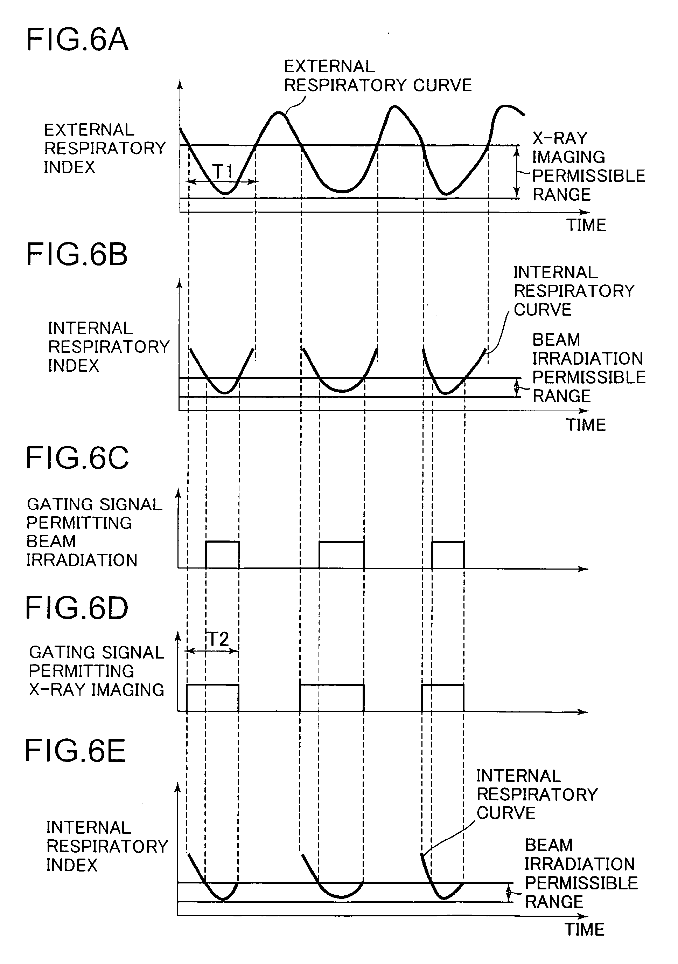

With reference now to FIGs. 5 and 6A to 6E, the configuration and operation of a radiotherapy system according to a second embodiment of the invention will be described. Note that the configuration of the radiotherapy system of the second embodiment is the same as the one shown in FIG. 1.

FIG. 5 is a flowchart illustrating the operation of the radiotherapy system of the second embodiment. Note that the same step numbers as used in FIG. 2 indicate the same operational steps.

FIGs. 6A to 6E are timing charts illustrating the operation of the radiotherapy system of the second embodiment. FIG. 6A shows the same external respiratory curve as shown in FIG. 3A. FIG. 6B shows the same internal respiratory curves as shown in FIG. 4A. FIG. 6C shows the same gating signals permitting beam irradiation as shown in FIG. 4B. FIG. 6D shows gating signals permitting x-ray imaging according to the second embodiment.

In Step S100, the operator starts up the external respiration monitor 300 to start the monitoring of an external respiratory index of the patient 130. The monitored external respiratory index is input to the x-ray imaging gating device 310.

In Step S200, the operator operates the x-ray imaging gating device 310 to set an x-ray imaging permissible range for the external respiratory index acquired by the external respiration monitor 300. The x-ray imaging gating device 310 is provided with means for the operator setting an x-ray imaging permissible range for the external respiratory index the x-ray imaging gating device 310 receives.

In Step S210, the x-ray imaging gating device 310 sends gating signals permitting x-ray imaging to the x-ray imaging controller 220.

More specifically, the x-ray imaging gating device 310 sends such gating signals permitting x-ray imaging as shown in FIG. 3B to the x-ray imaging controller 220 while the external respiratory index is within the x-ray imaging permissible range. Similar to the first embodiment, the x-ray imaging permissible range for the external respiratory index is input by the operator into the x-ray imaging gating device 310 by the operator referring to an external respiratory curve (FIG. 6A) displayed by the x-ray imaging gating device 310.

In Step S300A, the operator instructs the x-ray imaging controller 220 to start x-ray imaging in "normal mode." Similar to Step S300 of FIG. 2, normal mode is the mode in which the x-ray imaging controller 220 acquires x-ray images intermittently while the x-ray imaging controller 220 receives the gating signals permitting x-ray imaging from the x-ray imaging gating device 310.

In Step S400, the internal respiratory index acquisition device 400 analyzes the x-ray images acquired by the x-ray imaging controller 220 to obtain an internal respiratory index. The internal respiratory index is obtained from the positions of irradiation targets, bones, diaphragm, or implanted markers. The obtained internal respiratory index is sent to the irradiation gating device 410.

As stated above, x-ray images are acquired intermittently. Thus, when an analysis of the internal respiratory index needs to be followed by the next analysis without discontinuity at some later time, the radiotherapy system of the second embodiment can also be provided with a mechanism for storing the on/off states of the last gating signal on a memory and resuming analysis from the last on or off state of that signal.

In Step S500, the operator inputs into the irradiation gating device 410 a beam irradiation permissible range for the internal respiratory index by referring to its internal respiratory curves displayed by the irradiation gating device 410. Similar to the x-ray imaging gating device 310 having means for setting an x-ray imaging permissible range for the external respiratory index, the irradiation gating device 410 is provided with means for accepting the input of a gating condition for treatment beam irradiation. As shown in FIG. 6B, the internal respiratory curves are discontinuous since x-ray imaging is gated.

In Step S510, the irradiation gating device 410 sends such gating signals permitting beam irradiation as shown in FIG. 6C through the irradiation controller 500 to the beam extraction controller 140 while the internal respiratory index is within the beam irradiation permissible range.

In Step S520, the operator switches the mode of the x-ray imaging controller 220 from "normal mode" to "irradiation-synchronous mode." "Irradiation-synchronuous mode" is the mode in which x-ray imaging is permitted by such gating signals of x-ray imaging permission as shown in FIG. 6D that are turned off at the same time as the gating signals permitting beam irradiation that are shown in FIG. 6C are turned off.

The x-ray imaging permissible period T2 of FIG. 6D (radiation-synchronous mode of Step S520) is shorter than the x-ray imaging permissible period T1 of FIG. 6A (normal mode of Step S300A).

With the above steps, respiratory-gated irradiation becomes ready, which is based on the gating signals permitting beam irradiation that are synchronous with the beam irradiation permissible periods of the internal respiratory index.

In Step S600, the operator instructs the irradiation controller 500 to start treatment.

In Step S400A, the internal respiratory index acquisition device 400 analyzes x-ray images acquired by the x-ray imaging controller 220 during "irradiation-synchronous mode" to obtain the internal respiratory index again. FIG. 6E shows examples of internal respiratory curves obtained during "irradiation-synchronous mode."

In Step S700, the irradiation controller 500 instructs the beam extraction controller 140 to stop the beam extraction after a given dose of irradiation as specified by the treatment planning device 520.

Finally, in Step S800, the irradiation controller 500 instructs the x-ray imaging controller 220 to stop the x-ray imaging.

As stated above, the radiotherapy system of the second embodiment is designed to perform gated x-ray imaging by sending gating signals permitting x-ray imaging to the x-ray imaging controller 220 such that the x-ray imaging is turned off at the same time as gating signals permitting beam irradiation are turned off. Thus, the system is capable of acquiring internal respiratory waves or curves necessary for the gating control of beam irradiation with even less x-ray exposure.

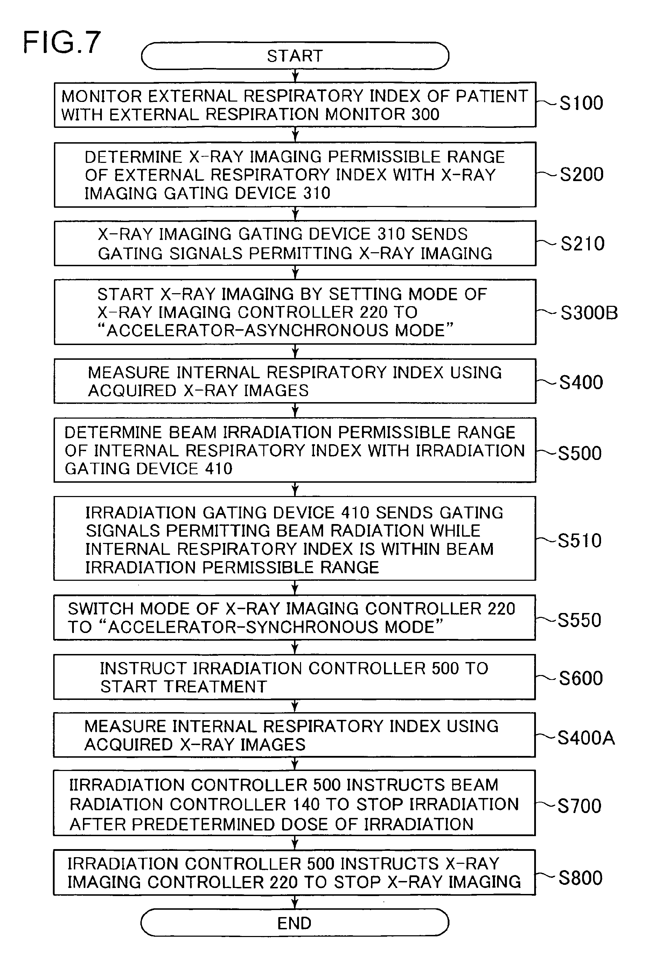

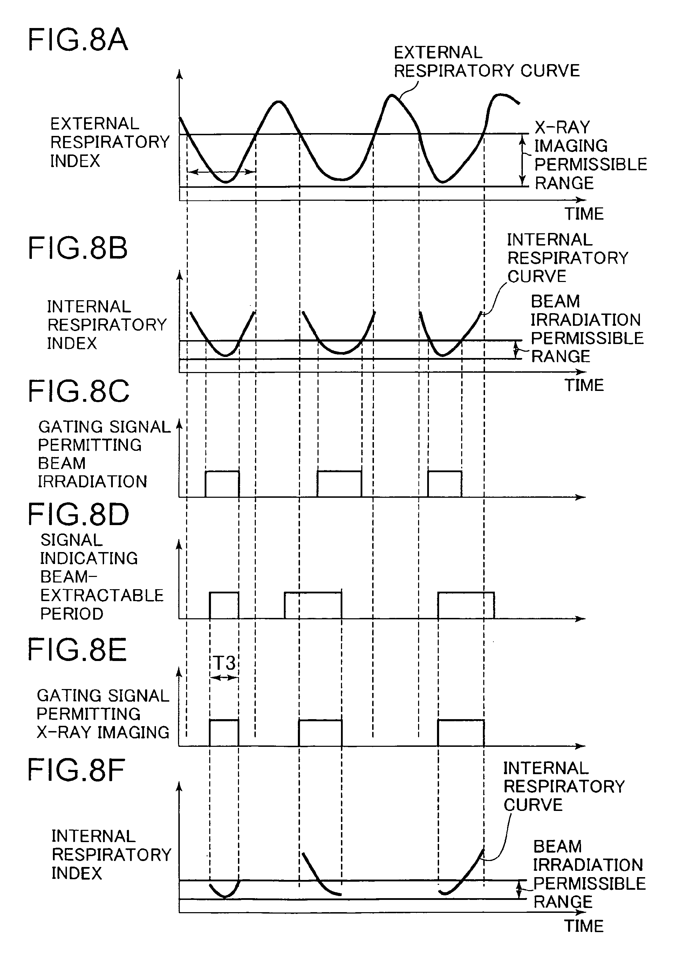

With reference now to FIGs. 7 and 8A to 8F, the configuration and operation of a radiotherapy system according to a third embodiment of the invention will be described. Note that the configuration of the radiotherapy system of the third embodiment is the same as the one shown in FIG. 1.

FIG. 7 is a flowchart illustrating the operation of the radiotherapy system of the third embodiment. Note that the same step numbers as used in FIGs. 2 and 5 indicate the same operational steps.

FIGs. 8A to 8F are timing charts illustrating the operation of the radiotherapy system of the third embodiment. FIGs. 8A to 8C are the same as FIGs. 5A to 5C, respectively. FIG. 8D shows signals indicative of beam-extractable periods. FIG. 8E shows gating signals permitting x-ray imaging according to the third embodiment. FIG. 8F shows internal respiratory curves according to the third embodiment.

In Step S100, the operator starts up the external respiration monitor 300 to start the monitoring of an external respiratory index of the patient 130. The monitored external respiratory index is input to the x-ray imaging gating device 310.

In Step S200, the operator operates the x-ray imaging gating device 310 to set an x-ray imaging permissible range for the external respiratory index acquired by the external respiration monitor 300. The x-ray imaging gating device 310 is provided with means for the operator setting an x-ray imaging permissible range for the external respiratory index the x-ray imaging gating device 310 receives.

In Step S210, the x-ray imaging gating device 310 sends gating signals permitting x-ray imaging to the x-ray imaging controller 220.

More specifically, the x-ray imaging gating device 310 sends such gating signals permitting x-ray imaging as shown in FIG. 3B to the x-ray imaging controller 220 while the external respiratory index is within the x-ray imaging permissible range. Similar to the first embodiment, the x-ray imaging permissible range for the external respiratory index is input by the operator into the x-ray imaging gating device 310 by the operator referring to an external respiratory curve (FIG. 8A) displayed by the x-ray imaging gating device 310.

In Step S300B, the operator instructs the x-ray imaging controller 220 to start x-ray imaging in "accelerator-asynchronous mode." Similar to Step S300 of FIG. 2 and normal mode, "accelerator-asynchronous mode" is the mode in which the x-ray imaging controller 220 acquires x-ray images intermittently while the x-ray imaging controller 220 receives the gating signals permitting x-ray imaging from the x-ray imaging gating device 310.

In Step S400, the internal respiratory index acquisition device 400 analyzes the x-ray images acquired by the x-ray imaging controller 220 to obtain an internal respiratory index. The internal respiratory index is obtained from the positions of irradiation targets, bones, diaphragm, or implanted markers. The obtained internal respiratory index is sent to the irradiation gating device 410.

As stated above, x-ray images are acquired intermittently. Thus, when an analysis of the internal respiratory index needs to be followed by the next analysis without discontinuity at some later time, the radiotherapy system of the third embodiment can also be provided with a mechanism for storing the on/off states of the last gating signal on a memory and resuming analysis from the last on or off state of that signal.

In Step S500, the operator inputs into the irradiation gating device 410 a beam irradiation permissible range for the internal respiratory index by referring to its internal respiratory curves displayed by the irradiation gating device 410. Similar to the x-ray imaging gating device 310 having means for setting an x-ray imaging permissible range for the external respiratory index, the irradiation gating device 410 is provided with means for accepting the input of a gating condition for treatment beam irradiation. As shown in FIG. 8B, the internal respiratory curves are discontinuous since x-ray imaging is gated.

In Step S500, the irradiation gating device 410 can be allowed to switch the mode of the x-ray imaging controller 220 from "accelerator-asynchronous mode" to "accelerator-synchronous mode" after the operator sets the beam irradiation permissible range. "Accelerator-synchronous mode" is the mode in which the x-ray imaging controller 220 acquires an x-ray image when the beam accelerator 100 is capable of radiating or extracting a treatment beam and the external respiratory index is within the x-ray imaging permissible range. In this case, when the beam-extractable time precedes a gating signal permitting x-ray imaging, x-ray imaging can be performed prior to that gating signal by a predetermined amount of time.

In Step S510, the irradiation gating device 410 sends such gating signals permitting beam irradiation as shown in FIG. 8C through the irradiation controller 500 to the beam extraction controller 140 while the internal respiratory index is within the beam irradiation permissible range.

In Step S550, the operator switches the mode of the x-ray imaging controller 220 from "accelerator-asynchronous mode" to "accelerator-synchronous mode." As stated briefly above, "accelerator-synchronous mode" is the mode in which x-ray imaging is permitted by such gating signals of x-ray imaging permission as shown in FIG. 8E that coincide with the beam-extractable periods of FIG. 8D while the external respiratory index of FIG. 8A is within the x-ray imaging permissible range. The extractability of a treatment beam from the accelerator 100 is judged by a signal indicative of a beam-extractable period which is sent from the beam extraction controller 140.

The x-ray imaging permissible period T3 of FIG. 8E (accelerator-synchronous mode of Step S550) is shorter than the x-ray imaging permissible period T1 of FIG. 8A (accelerator-asynchronous mode of Step S300B).

With the above steps, respiratory-gated irradiation becomes ready, which is based on the gating signals permitting beam irradiation that are synchronous with the beam irradiation permissible periods of the internal respiratory index.

In Step S600, the operator instructs the irradiation controller 500 to start treatment. The irradiation controller 500 then starts to send to the x-ray imaging controller 220 such signals indicative of beam-extractable periods as shown in FIG. 8D. The extractability of treatment beams from the accelerator 100 is judged by the above signals indicative of beam-extractable periods that are received by the irradiation controller 500 from the beam extraction controller 140. The beam extraction controller 140 judges the extractability of a treatment beam based on the states of the accelerator 100 and the nozzle 110 and sends those signals indicative of beam-extractable periods to the irradiation controller 500.

In Step S400A, the internal respiratory index acquisition device 400 analyzes x-ray images acquired by the x-ray imaging controller 220 during "acceleration-synchronous mode" to obtain the internal respiratory index again.

In Step S700, the irradiation controller 500 instructs the beam extraction controller 140 to stop the beam extraction after a given dose of irradiation as specified by the treatment planning device 520.

Finally, in Step S800, the irradiation controller 500 instructs the x-ray imaging controller 220 to stop the x-ray imaging.

As stated above, the radiotherapy system of the third embodiment is designed to perform gated x-ray imaging by sending gating signals permitting x-ray imaging to the x-ray imaging controller 220 in synchronization with signals indicative of beam-extractable periods. Thus, the system is capable of acquiring internal respiratory waves or curves necessary for the gating control of beam irradiation with far less x-ray exposure.

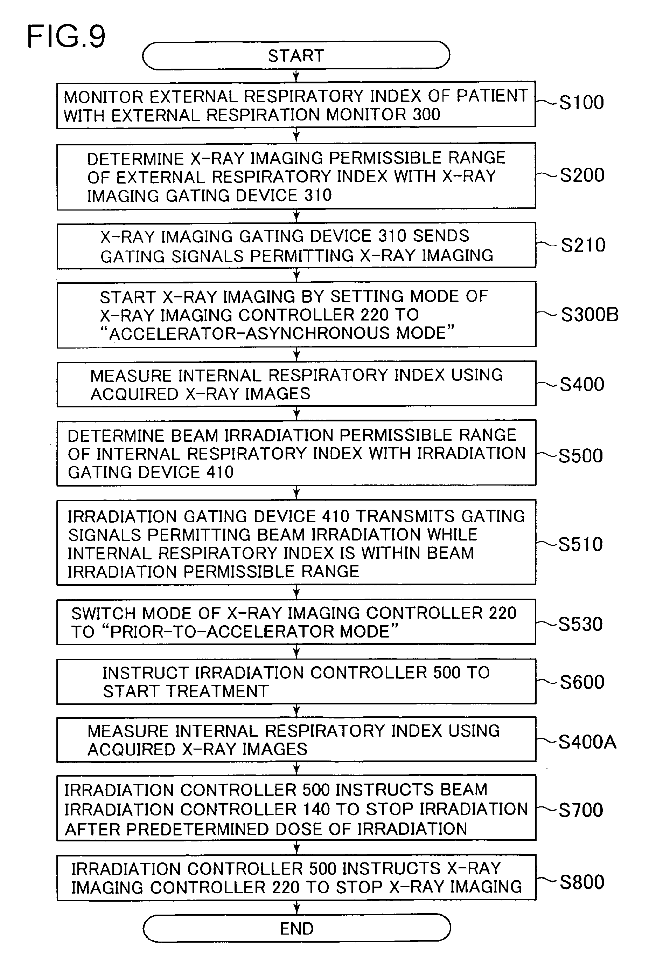

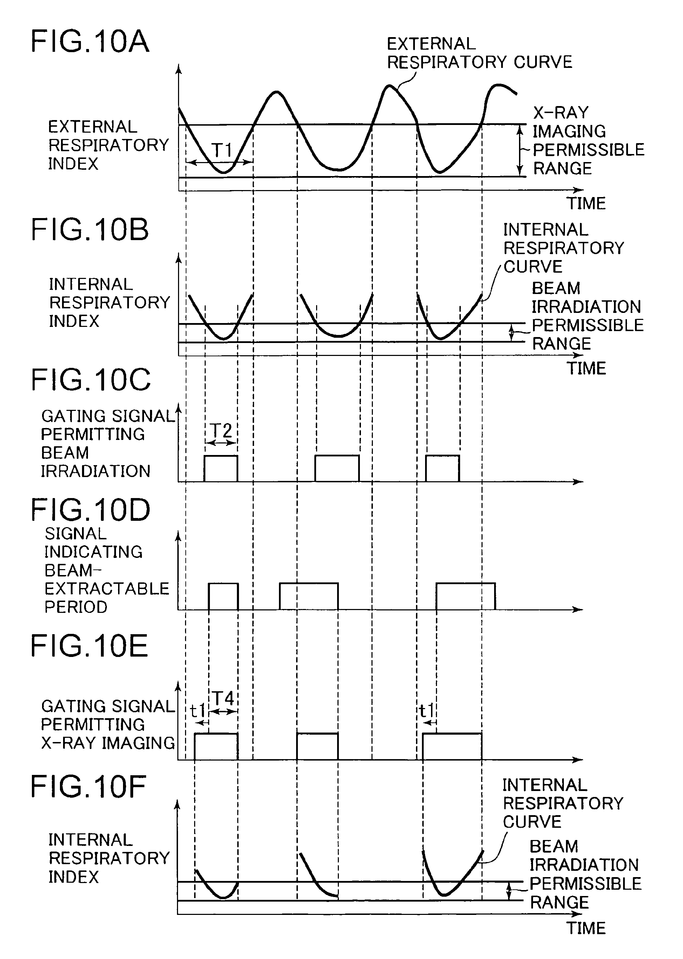

With reference now to FIGs. 9 and 10A to 10F, the configuration and operation of a radiotherapy system according to a fourth embodiment of the invention will be described. Note that the configuration of the radiotherapy system of the fourth embodiment is the same as the one shown in FIG. 1.

FIG. 9 is a flowchart illustrating the operation of the radiotherapy system of the fourth embodiment. Note that the same step numbers as used in FIGs. 2, 5, and 7 indicate the same operational steps.

FIGs. 10A to 10F are timing charts illustrating the operation of the radiotherapy system of the fourth embodiment. FIGs. 10A to 10D are the same as FIGs. 8A to 8D, respectively. FIG. 10E shows gating signals permitting x-ray imaging according to the fourth embodiment. FIG. 10F shows internal respiratory curves according to the fourth embodiment.

In Step S100, the operator starts up the external respiration monitor 300 to start the monitoring of an external respiratory index of the patient 130. The monitored external respiratory index is input to the x-ray imaging gating device 310.

In Step S200, the operator operates the x-ray imaging gating device 310 to set an x-ray imaging permissible range for the external respiratory index acquired by the external respiration monitor 300. The x-ray imaging gating device 310 is provided with means for the operator setting an x-ray imaging permissible range for the external respiratory index the x-ray imaging gating device 310 receives.

In Step S210, the x-ray imaging gating device 310 sends gating signals permitting x-ray imaging to the x-ray imaging controller 220.

More specifically, the x-ray imaging gating device 310 sends such gating signals permitting x-ray imaging as shown in FIG. 3B to the x-ray imaging controller 220 while the external respiratory index is within the x-ray imaging permissible range. Similar to the first embodiment, the x-ray imaging permissible range for the external respiratory index is input by the operator into the x-ray imaging gating device 310 by the operator referring to an external respiratory curve (FIG. 10A) displayed by the x-ray imaging gating device 310.

In Step S300B, the operator instructs the x-ray imaging controller 220 to start x-ray imaging in "accelerator-asynchronous mode." As stated in Step S300B of FIG. 7, "accelerator-asynchronous mode" is the mode in which the x-ray imaging controller 220 acquires x-ray images intermittently while the x-ray imaging controller 220 receives the gating signals permitting x-ray imaging from the x-ray imaging gating device 310.

In Step S400, the internal respiratory index acquisition device 400 analyzes the x-ray images acquired by the x-ray imaging controller 220 to obtain an internal respiratory index. The internal respiratory index is obtained from the positions of irradiation targets, bones, diaphragm, or implanted markers. The obtained internal respiratory index is sent to the irradiation gating device 410.

As stated above, x-ray images are acquired intermittently. Thus, when an analysis of the internal respiratory index needs to be followed by the next analysis without discontinuity at some later time, the radiotherapy system of the fourth embodiment can also be provided with a mechanism for storing the on/off states of the last gating signal on a memory and resuming analysis from the last on or off state of that signal.

In Step S500, the operator inputs into the irradiation gating device 410 a beam irradiation permissible range for the internal respiratory index by referring to its internal respiratory curves displayed by the irradiation gating device 410. Similar to the x-ray imaging gating device 310 having means for setting an x-ray imaging permissible range for the external respiratory index, the irradiation gating device 410 is provided with means for accepting the input of a gating condition for treatment beam irradiation. As shown in FIG. 10B, the internal respiratory curves are discontinuous since x-ray imaging is gated.

In Step S500, the irradiation gating device 410 can be allowed to switch the mode of the x-ray imaging controller 220 from "accelerator-asynchronous mode" to "accelerator-synchronous mode" after the operator sets the beam irradiation permissible range. As stated above, accelerator-synchronous mode is the mode in which the x-ray imaging controller 220 acquires an x-ray image when the beam accelerator 100 is capable of radiating or extracting a treatment beam and the external respiratory index is within the x-ray imaging permissible range. In this case, when the beam-extractable time precedes a gating signal permitting x-ray imaging, x-ray imaging can be performed prior to that gating signal by a predetermined amount of time.

In Step S510, the irradiation gating device 410 sends such gating signals permitting beam irradiation as shown in FIG. 10C through the irradiation controller 500 to the beam extraction controller 140 while the internal respiratory index is within the beam irradiation permissible range.

In Step S530, the operator switches the mode of the x-ray imaging controller 220 from "accelerator-asynchronous mode" to "prior-to-accelerator mode." "Prior-to-accelerator mode" is the mode in which x-ray imaging is permitted by such gating signals of x-ray imaging permission as shown in FIG. 10E that are turned on prior to the beam-extractable periods of FIG. 10D by time T1 and turned off at the same time as the end of the beam-extractable periods while the external respiratory index of FIG. 10A is within the x-ray imaging permissible range.

The x-ray imaging permissible period (t1 + T4) of FIG. 10E (prior-to-acceleration mode of Step S530) is shorter than the x-ray imaging permissible period T1 of FIG. 10A (accelerator-asynchronous mode of Step S300B) and also shorter than the x-ray imaging permissible period T2 of FIG. 6D. Although the x-ray imaging permissible period (t1 + T4) is longer than the x-ray imaging permissible period T3 of FIG. 8E (accelerator-synchronous mode), the internal respiratory index can be monitored for a slightly longer amount of time.

With the above steps, respiratory-gated irradiation becomes ready, which is based on the gating signals permitting beam irradiation that are synchronous with the beam irradiation permissible periods of the internal respiratory index.

In Step S600, the operator instructs the irradiation controller 500 to start treatment. The irradiation controller 500 then starts to send to the x-ray imaging controller 220 such signals indicative of beam-extractable periods as shown in FIG. 10D. The extractability of treatment beams from the accelerator 100 is judged by the above signals indicative of beam-extractable periods that are received by the irradiation controller 500 from the beam extraction controller 140. The beam extraction controller 140 judges the extractability of a treatment beam based on the states of the accelerator 100 and the nozzle 110 and sends those signals indicative of beam-extractable periods to the irradiation controller 500.

In Step S400A, the internal respiratory index acquisition device 400 analyzes x-ray images acquired by the x-ray imaging controller 220 during "prior-to-accelerator mode" to obtain the internal respiratory index again.

In Step S700, the irradiation controller 500 instructs the beam extraction controller 140 to stop the beam extraction after a given dose of irradiation as specified by the treatment planning device 520.

Finally, in Step S800, the irradiation controller 500 instructs the x-ray imaging controller 220 to stop the x-ray imaging.

As stated above, the radiotherapy system of the fourth embodiment is designed to perform gated x-ray imaging by sending gating signals permitting x-ray imaging to the x-ray imaging controller 220 in synchronization with and prior to signals indicative of beam-extractable periods. Thus, the system is capable of acquiring internal respiratory waves or curves necessary for the gating control of beam irradiation with far less x-ray exposure.

## Claims

1. A radiotherapy system comprising:
external-respiration observation means for externally monitoring an external respiratory index of a patient;
internal-respiration observation means for acquiring x-ray images of the patient and monitoring an internal respiratory index of the patient using the positions of internal body structures indicated by the acquired x-ray images;
imaging gating means for gating the x-ray imaging performed by the internal-respiration observation means with the use of the external respiratory index monitored by the external-respiration observation means such that the x-ray imaging is performed while the external respiratory index is within a predetermined range; and
beam irradiation gating means for gating the irradiation of a treatment beam with the use of the internal respiratory index monitored by the internal-respiration observation means.

2. The radiotherapy system defined in claim 1, wherein the internal-respiration observation means includes x-ray imaging control means for instructing the internal-respiration observation means to stop the x-ray imaging when a gating signal permitting treatment-beam irradiation which is generated by the beam irradiation gating means is turned off.

3. The radiotherapy system defined in claim 1, wherein the internal-respiration observation means includes x-ray imaging control means for permitting the internal-respiration observation means to perform the x-ray imaging while a treatment beam can be radiated.

4. The radiotherapy system defined in claim 1, wherein the internal-respiration observation means includes x-ray imaging control means for permitting the internal-respiration observation means to perform the x-ray imaging prior to a period during which a treatment beam can be radiated.

5. The radiotherapy system defined in claim 1, wherein the beam irradiation gating means stores a condition for radiating a treatment beam so that the beam irradiation gating means can use the condition for later treatments.
